# EUROPEAN PATENT SPECIFICATION

(11) **EP 0 335 652 B1**
(45) Date of publication and mention of the grant of the patent: **01.09.1993**
(21) Application number: 89303036.1
(22) Date of filing: 28.03.1989
(51) Int. Cl.: A61F 5/445, A61F 5/442

(54) **Devices for use by ostomy patients**
Vorrichtungen zur Verwendung durch Ostomiepatienten
Dispositifs destinés à l'usage des patients ostomiques

(30) Priority: 29.03.1988 GB 8807378
(43) Date of publication of application: 04.10.1989
(73) Proprietor: Knox, Patrick Robert William, Abbeylands Isle of Man (GB); Holt, David Arthur, Douglas Isle of Man (GB); Dryden, Harry Peter, Baldrine Isle of Man (GB)
(72) Inventor: Knox, Patrick Robert William, Abbeylands Isle of Man (GB); Holt, David Arthur, Douglas Isle of Man (GB); Dryden, Harry Peter, Baldrine Isle of Man (GB)
(74) Representative: Jones, William

(56) References cited:
- US-A- 2 639 711
- US-A- 2 813 530
- US-A- 4 429 424

## Description

### Field of the Invention

This invention relates to devices for use by ostomy patients, in particular patients who have undergone ileosostomy or urostomy surgery.

### Related Application

In UK specification No 2 206 274A published on 5th January 1989, there is described a storage and carrier tray for use by ostomy patients, which tray includes compartments within which are disposed those items required for changing an ostomy bag, the tray being provided with means whereby it can be supported on the body of the user.

It is an object of the present invention to provide a device for use by ostomy patients which is less expensive to produce than this known tray and whose contents can be collapsed into a disposal bag by the user and hence more easily disposed of.

### Summary of the Invention

According to the present invention there is provided a device for use by ostomy patients, which device comprises a frame adapted for the lap of a seated patient, a chute depending from the frame and intended, in use, to extend between the legs or thighs of the patient, an opening in said frame and communicating with the chute whereby the contents of an ostomy pouch can be deposited in and flow down the chute, of respective first and second portions of material, the one connecting the chute detachably to the frame, the other depending from the first portion and constituting or forming part of the chute; and with means, attaching to or forming part of said portions and manipulable therewith by the patient in use to collapse the portions into a disposal bag; both the said portions being constituted by a single and relatively flexible sheet of material in which corner-apertures fit detachably over respective posts on the frame; characterized by the provision of a flexible tape which is connected at one end to the lower end of the chute and has its other end accessible from the level of the frame. The flexible tape is preferably arranged so that it extends inside and alongside the chute. A scissor-formation member (Figure 10) may be provided to enable the tape to be pulled upwards, thereby collapsing the flexible chute portion, in use.

In other embodiments, the first portion may be basin-shaped and made from relatively rigid material whilst the second portion takes the form of a flexible sleeve having open upper and lower ends; or the frame itself may comprise a flat and relatively rigid sheet with the chute portions fitting in an opening in that sheet.

### Brief Description of the Drawings

Figure 1 is a plan view of a first form of ostomy device.
Figure 2 is a side view of the device shown in Figure 1.
Figure 3 is a plan view of a second form of ostomy device.
Figure 4 is a side view of the device shown in Figure 3.
Figure 5 is a plan view of a third form of ostomy device,
Figure 6 is a side view of the device shown in Figure 5,
Figure 7 is a front view of a case for transporting the device of Figure 5,
Figure 8 is an end view, Figure 9 a plan view, of Figure 7;
Figure 10 shows a fourth embodiment in perspective.

### Description of the Preferred Embodiments

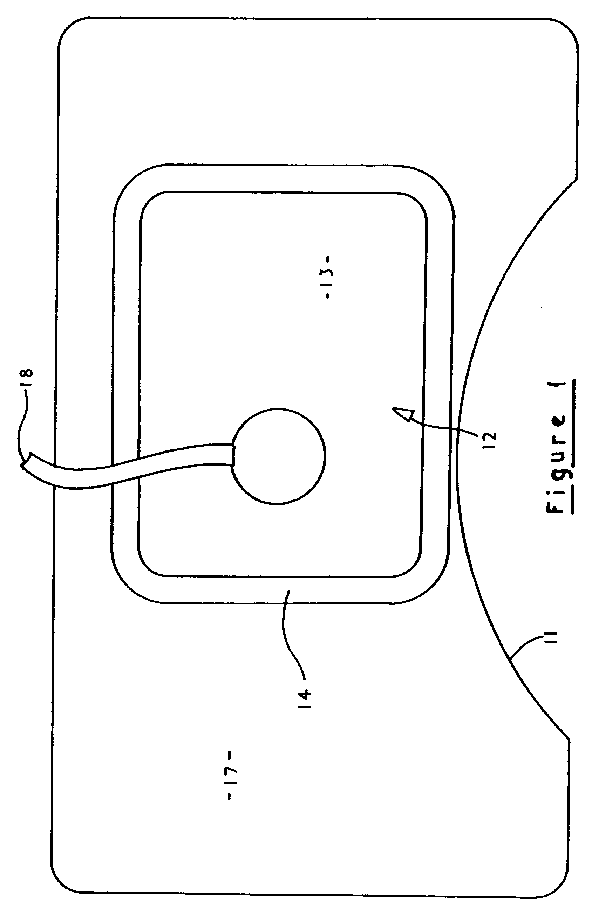

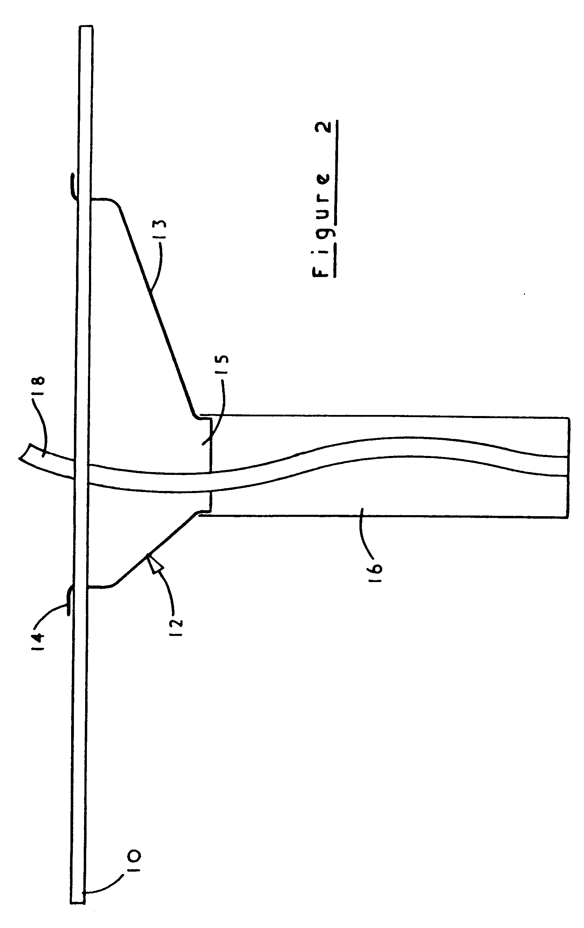

The device shown in Figures 1 and 2 includes a flat sheet 10 which is formed from a synthetic plastics material and which has sufficient rigidity and strength to enable it to function as a support tray. The sheet 10 is of generally rectangular form as shown, with rounded corners, and with an arcuate rebate 11 formed in one of the longer edges of the sheet. The support tray provided by the sheet 10 is intended to be placed on the lap of an ostomy patient when seated on the toilet and the purpose of the rebate 11 is accordingly to enable the support tray to fit closely against the trunk of the patient with the patient's stomach fitting within the rebate 11.

The plastics sheet 10 is formed with a through aperture into which a disposable drainage module 12 is fitted. The disposable drainage module includes a first part 13 which is in the form of a plastics moulding; the plastics moulding is relatively thin-walled and is shaped generally in the form of a basin with a peripheral lip 14 which sits on the surrounds of the through opening in the sheet 10 so that the drainage module 12 is seated removably within the opening in the sheet 10. The basin-like plastics moulding 13 has sloping side walls as shown in Figure 2 which lead to a discharge outlet 15 of generally cylindrical form to which is attached a sleeve 16 which is open at its lower end and which functions as a chute. The discharge outlet 15 is disposed generally centrally of the support tray defined by the plastics sheet 10 so that, when the tray is placed on the lap of the ostomy patient, the chute provided by the sleeve 16 extends downwardly between the legs or thighs of the patient. The opening in the sheet 10 within which the drainage module 12 is located is, however, offset as can be seen from Figures 1 and 2 so that a working area 17 is defined on one side of the basin provided by the plastics moulding 13, on which working area the patient can place those items which he requires to use during changing and emptying of an ostomy pouch. A flexible tape 18 is attached at its lower end to the open lower end of the sleeve 16 and the flexible tape 18 extends upwardly inside the chute, as shown in Figure 1, so that it can be grasped manually by the patient.

In use, upon completion of the emptying of the contents of an ostomy pouch into the basin and thence, via the chute into the toilet, the patient grasps the upper end of the flexible tape 18 and pulls the chute afforded by the flexible sleeve 16 into the confines of the basin afforded by the plastic moulding 13. The module 12 as a whole is then removed from the support tray afforded by the plastic sheet 10 and the module fitted into a coloured self-sealing disposal bag.

If the ostomy equipment has to be changed, the plain working surface afforded by the area 17 can be used in conjunction with, if desired, an additional sheet of plastics material which can be placed on top of the tray so as to cover the basin and to provide additional room on which to place the items required for changing an ostomy pouch.

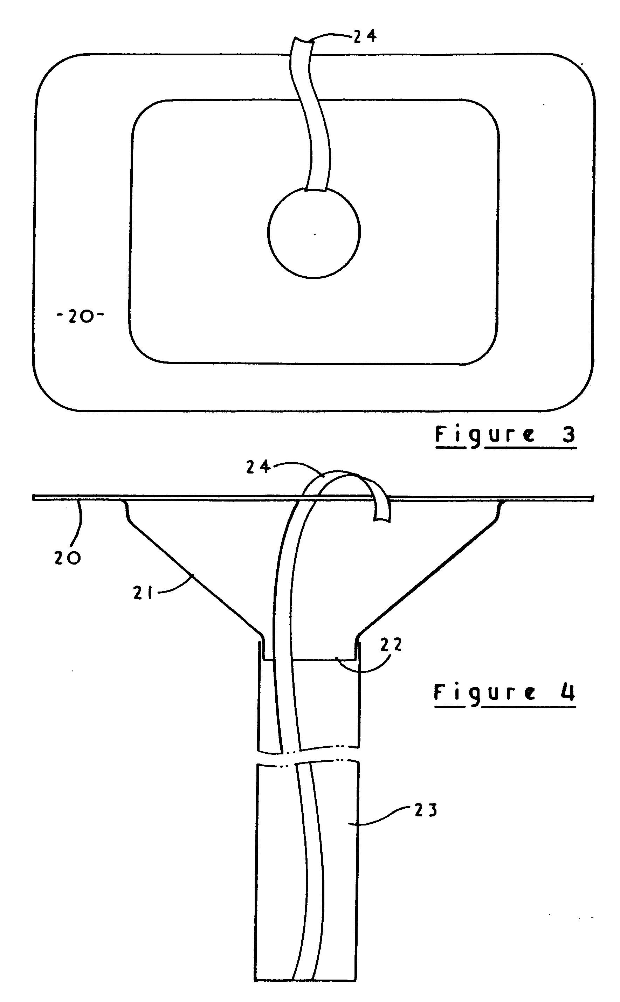

Turning next to Figures 3 and 4, these show a second form of the device which includes a one-piece moulding of a relatively thin-walled plastics material, which moulding includes a planar portion 20 intended to rest on the lap of the user of the device when seated on the toilet, and a basin-shaped portion 21 which has downwardly inclined side walls which terminate in a discharge outlet 22. The discharge outlet 22 is disposed centrally of the plastics moulding and has attached to it a flexible plastics sleeve 23 which provides a chute which is open at its lower end and to the inside of which a flexible tape 24 is attached.

The embodiment shown in Figures 3 and 4 differs from that of Figures 1 and 2 in that the device as a whole is intended to be disposable. The contents of an ostomy pouch are emptied into the portion 21 so as to pass into the chute and thence into the toilet; the tape 24 is pulled to draw the chute into the interior of the basin and then the disposable module as a whole is placed inside a coloured self-sealing disposal bag.

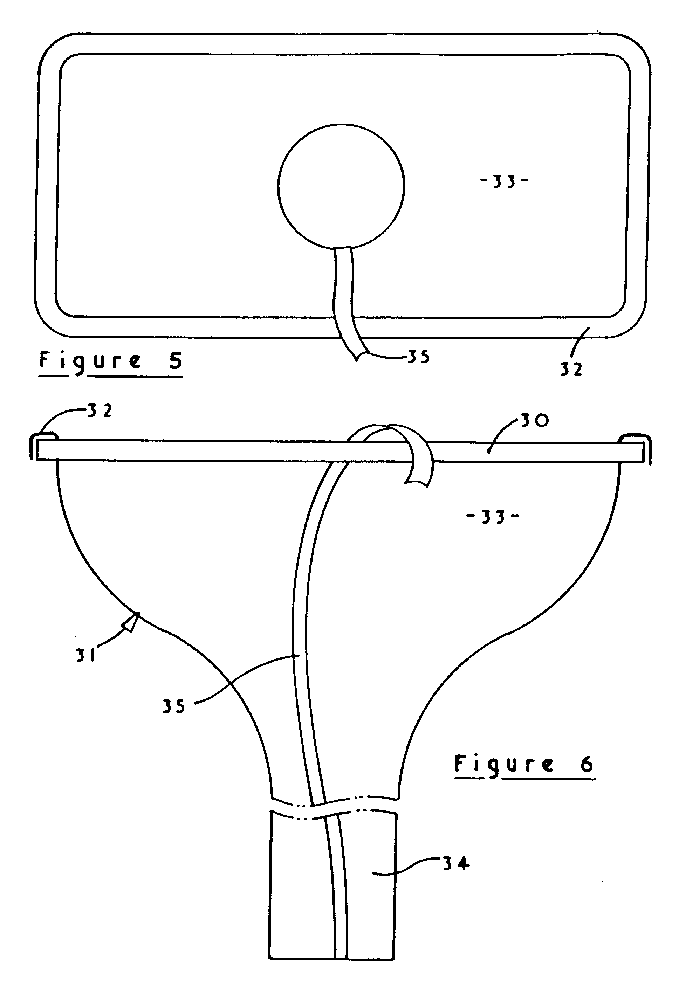

Turning next to Figures 5 and 6, these show a device which includes a rectangular frame 30 which is preferably formed of stainless steel but may be formed of a relatively rigid plastics material by a suitable moulding operation. The frame 30 functions as a support for a shaped flexible plastics sleeve 31, the sleeve 31 being provided at one end with a formation 32 which serves to fit over the frame 30 to hold the sleeve 31 resiliently in position carried by the frame 30. After use, the formation 32 can readily be detached from the frame 30 so that the used sleeve 31 can be placed within a coloured self-sealing disposal bag.

The sleeve 31 is shaped so that its upper portion 33 defines a basin corresponding to the embodiments shown in Figures 1 and 2 or Figures 3 and 4 and this upper portion then merges with a lower chute-like portion 34, the lower end of which is open and to the inside of which is attached a flexible tape 35. The mode of use of the device of Figures 5 and 6 will be readily apparent from the comments set out above.

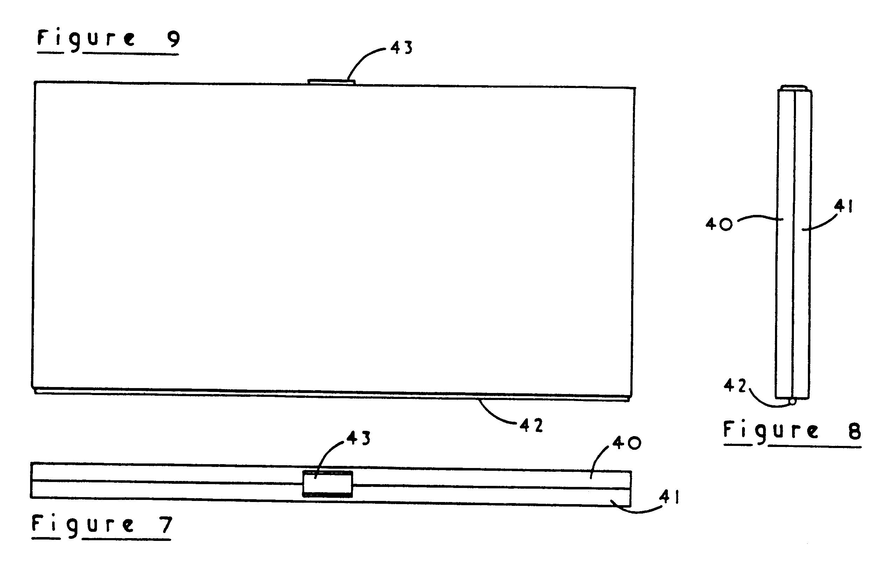

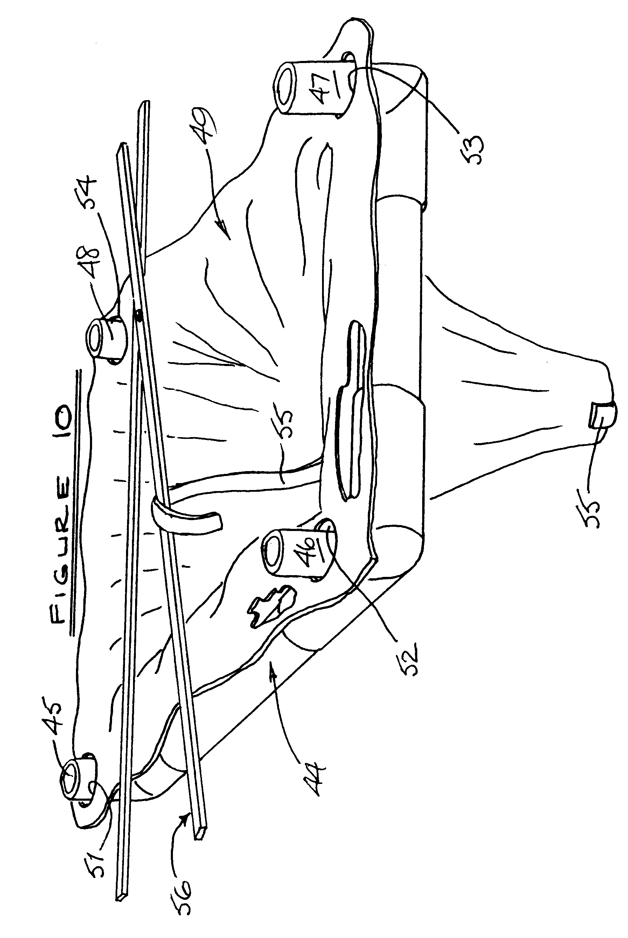

An advantage of the form of device shown in Figures 5 and 6 is that it can be folded, prior to use, into a generally flat condition in which transport thereof is facilitated. One form of case for use in transporting the device of Figures 5 and 6 is shown in Figures 7 to 9. The case comprises two halves 40 and 41 interconnected by a hinge 42 with a catch 43 which slideable between locking and release positions and which, in its locking position, serves to hold the two halves of the case together.

The case can be used not only to transport the device on Figures 5 and 6 but it can also be used to provide a working area on which items can be placed whilst changing or emptying an ostomy pouch. The case can also be used to contain the coloured disposal bag in which, after emptying a pouch, the draining module 30, 31 can be placed.

In Figure 10 a rectangular frame 44 is formed from "plug-in" rigid moulded plastics fittings and has upstanding corner pieces 45 through 48. A shaped flexible plastics sleeve 49 is formed with corner holes 51 through 54 whose spacing is identical to the relative spacing of the upstanding posts 45 through 48. The sleeve 49 is so sized and shaped that, when the holes 51 through 54 are fitted over their respective corner posts 45 through 48, the sleeve 49 forms a combined basin area and chute in much the same way as the sleeve 31 of the embodiment of Figures 5 and 6.

The now-familiar flexible tape 55 is secured to the open lower end of the chute and extends upwardly inside the chute to a scissor-formed hand-held member 56 over which the top end of the tape 55 runs. Member 56 enables tape 55 to be pulled, without fear of the top end of the tape falling inadvertently into the chute of the device, and the whole device is then disposable.

Alternatively, sleeve 49 can be detached from corner posts 45 through 48 and the sleeve, tape and (optionally) scissor-formation member 56 can be disposed of whilst frame 44 (and optionally member 56) are retained for the next usage with a supply of disposable sleeves 49.

Scissor-formation member 56 is long enough to span the opposite sides of frame 44, as illustrated, and rests on top of those frame sides so that neither the member 56 nor the upper end of the tape 55 should inadvertently fall into the chute before the user picks up member 56 to raise the chute for disposal after use.

In Figure 10, parts of the sheet 49 adjacent post 46 are cut away to show the construction of frame 44.

## Claims

1. A device for use by ostomy patients, which device comprises a frame (10; 20; 30; 44) adapted for the lap of a seated patient, a chute (16; 23; 34; 49) depending from the frame and intended, in use to extend between the legs or thighs of the patient; and an opening in said frame communicating with the chute whereby the contents of an ostomy pouch can be deposited in and flow down the chute; a chute transfer means comprising a first portion of material (12; 24; 33) which connects the chute detachably to the frame; a second portion (16; 23; 34; 49) which depends from the first portion and which constitutes or forms part of the chute; and means (18; 24; 35; 51 through 54 and 45 through 48) attaching to or forming part of said portions and manipulable therewith by the patient in use to collapse the portions into a disposal bag; and characterized by the feature that the last-mentioned means comprises a length of tape (18; 24; 35; 55) which is connected at its one end to the lower end of the chute and has its other end accessible from the level of the frame.

2. A device according to Claim 1 characterised by the feature that the said tape (18; 24; 35; 55) extends inside and alongside the chute wall.

3. A device according to any of the preceding Claims and characterised by the feature that the frame is in the form of a rectangular framework (30; 44) over which the said first portion (31; 49) of material fits.

4. A device according to any of Claim 1 and characterised by the feature that the frame comprises a flat sheet (10) having an opening into which the said first portion (12) fits.

5. A device according to any of the preceding Claims characterised by the feature that the chute comprises an upper part (13; 21) which is shaped in the form of a basin, and is formed of relatively rigid material, and a lower part (16; 23) which is in the form of a flexible sleeve having open upper and lower ends.

6. A device according to Claim 4 characterised by the feature that the said first and second portions of material are provided by a single appropriately shaped sheet of material (31; 49).

7. A device according to any of Claims 1 to 8 characterised by the feature that there is provided an elongate hand-held member (56) which is so sized as to span said frame (44) and so shaped as to enable the upper end of said length of tape (55) to engage and be retained by said member, whereby a user of the device can hand-lift the member (56) to pull upwards the tape (55).

8. A device according to Claim 7 characterised by the feature that the member (56) is a scissor-formation member.

## Patentansprüche

1. Vorrichtung zur Benutzung von Kolostomiepatienten, wobei die Vorrichtung einen Rahmen (10; 20; 30; 44) umfasst, der an den Schoss eines sitzenden Patienten angepasst ist, eine Rinne (16; 23; 34; 49), die von dem Rahmen herabhängt, und dazu bestimmt ist, sich während der Benutzung zwischen den Beinen oder Oberschenkeln des Patienten zu erstrecken; und eine Öffnung in dem Rahmen, die mit der Rinne in Verbindung steht, wobei der Inhalt eines Kolostomiebeutels darin abgelagert werden kann, und die Rinne herunterfliessen kann; ein Rinnenübertragungsmittel, das einen ersten Materialteil (12; 21; 33) umfasst, der die Rinne abnehmbar mit dem Rahmen verbindet; einen zweiten Teil (16; 23; 34; 49), der von dem ersten Teil herabhängt, und das die Rinne oder einen Teil davon bildet oder formt; und ein Mittel (18; 24; 35; 51 bis 54 und 45 bis 48), das an die Teile angebracht wird, oder einen Teil davon bildet, und damit von dem Patienten während der Benutzung gehandhabt werden kann, um die Teile in einen Wegwerfbeutel zusammenzufalten; und gekennzeichnet durch das Merkmal, dass das letztgenannte Mittel eine Bandlänge (18; 24; 35; 55) umfasst, die an ihrem einen Ende an das untere Ende der Rinne angeschlossen ist, und deren anderes Ende von der Ebene des Rahmens erreicht werden kann.

2. Vorrichtung nach Anspruch 1, gekennzeichnet durch das Merkmal, dass sich das Band (18; 24; 35; 55) innerhalb und entlang der Rinne erstreckt.

3. Vorrichtung nach einem der vorhergehenden Ansprüche, gekennzeichnet durch das Merkmal, dass der Rahmen in der Gestalt eines rechteckigen Gerüstes (30; 44) ist, über das der erste Materialteil passt.

4. Vorrichtung nach Anspruch 1, gekennzeichnet durch das Merkmal, dass der Rahmen ein flaches Blatt (10) mit einer Öffnung umfasst, in welche der erste Teil passt.

5. Vorrichtung nach einem der vorhergehenden Ansprüche, gekennzeichnet durch das Merkmal, dass die Rinne einen oberen Teil (13; 21) umfasst, der in der Gestalt einer Schale geformt ist, und von relativ festem Material gebildet ist, und einen unteren Teil (16; 23) umfasst, der in Gestalt eines flexiblen Ärmels mit offenen oberen und unteren Enden gebildet ist.

6. Vorrichtung nach Anspruch 4, gekennzeichnet durch das Merkmal, dass die ersten und zweiten Materialteile von einem einzelnen, geeignet gestalteten Materialblatt (31; 49) geliefert werden.

7. Vorrichtung nach einem der Ansprüche 1 bis 6, gekennzeichnet durch das Merkmal, dass ein langgestrecktes, von Hand gehaltenes Glied (56) vorgesehen ist, mit einer Grösse, die den Rahmen (44) überspannt, und derart gestaltet ist, dass es das obere Ende der Bandlänge (55) befähigt, in das Glied einzugreifen und davon zurückgehalten zu werden, wobei eine Benutzungsperson der Vorrichtung das Glied (56) mit der Hand anheben kann, um das Band nach oben zu ziehen.

8. Vorrichtung nach Anspruch 7, gekennzeichnet durch das Merkmal, dass das Glied (56) ein Glied in Gestalt einer Schere ist.

## Revendications

1. Dispositif prévu à l'intention des patients ayant subi une colostomie, comportant un cadre (10;20;30;44) adapté pour le patient en position assise, une goulotte (16; 23; 34; 49) montée sur le cadre et prévue à l'emploi pour loger entre les jambes ou les cuisses du patient; et une ouverture dans ledit cadre en communication avec la goulotte permettant de déposer et évacuer le contenu d'un sac de colostomie dans la goulotte; un moyen de transfert de la goulotte comportant un premier élément de matériau (12; 21; 33) raccordant la goulotte au cadre de façon amovible; un deuxième élément (16; 23; 34; 49) fonction du premier élément et constituant ou formant un élément de la goulotte; et des moyens (18; 24; 35; 51 à 54 et 45 à 48) attachés à ou formant corps avec lesdits éléments et prévus pour la manipulation par le patient de façon à escamoter ces éléments dans un sac jetable; et caractérisé par le fait que ces dits derniers moyens comporte une longueur de bande (18; 24; 35; 55) dont une extrémité est attachée à l'extrémité inférieure de la goulotte et l'autre extrémité est accessible depuis le niveau dudit cadre.

2. Dispositif selon la revendication 1 caractérisé par le fait que ladite bande (18; 24; 35; 55) est disposée à l'intérieur et le long de la paroi de la goulotte.

3. Dispositif selon l'une ou l'autre des revendications précédentes caractérisé par le fait que le cadre est sous forme de bâti rectangulaire (30; 44) sur lequel se pose ledit premier élément de matériau (31; 49).

4. Dispositif selon la revendication 1 et caractérisé par le fait que le cadre comporte une feuille plate (10) ayant une ouverture qui admet le premier élément (12).

5. Dispositif selon l'une ou l'autre des revendications précédentes caractérisé par le fait que la goulotte comporte un élément supérieur (13; 21) sous forme de cuvette, et en matériau relativement rigide, ainsi qu'un élément inférieur (16; 23) en forme de manchon flexible ouvert à l'extrémité supérieure et inférieure.

6. Dispositif selon la revendication 4 caractérisé par le fait que lesdits éléments de matériau sont prévus sous forme de feuille unique de matériau façonnée de manière appropriée (31; 49).

7. Dispositif selon l'une des revendications 1 à 6 caractérisé par le fait qu'il est prévu un élément manuel allongé (56) dont les dimensions sont telles à porter de chaque côté du cadre (44) et la forme est telle à permettre audit élément d'engager et de retenir l'extrémité supérieure de la longueur de bande (55), l'utilisateur dudit dispositif étant alors en mesure de relever ledit élément (56) à la main pour tirer et relever la bande (55).

8. Dispositif selon la revendication 7 caractérisé par le fait que ledit élément (56) forme un genre de ciseau.
